(19) Europäisches Patentamt
European Patent Office
Office européen des brevets

(11) Veröffentlichungsnummer : **0 034 784**
**B1**

(12) # EUROPÄISCHE PATENTSCHRIFT

(45) Veröffentlichungstag der Patentschrift : **25.07.84**

(21) Anmeldenummer : **81101056.0**

(22) Anmeldetag : **14.02.81**

(51) Int. Cl.³ : **C 07 D211/46**, A 61 K 31/445, A 23 K 1/16, C 07 D401/12// C07C149/34 ,(C07D401/12, 211/46, 207/40)

(54) **Derivate des 3,4,5-Trihydroxypiperidins, Verfahren zu ihrer Herstellung und ihre Verwendung als Arzneimittel sowie in der Tierernährung.**

(30) Priorität : **26.02.80 DE 3007078**

(43) Veröffentlichungstag der Anmeldung : **02.09.81 Patentblatt 81/35**

(45) Bekanntmachung des Hinweises auf die Patenterteilung : **25.07.84 Patentblatt 84/30**

(84) Benannte Vertragsstaaten : **AT BE CH DE FR GB IT LI LU NL SE**

(56) Entgegenhaltungen : **GB-A- 2 020 278**

**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Junge, Bodo, Dr.**
**Wilkhausstrasse 123**
**D-5600 Wuppertal 1 (DE)**
Erfinder : **Stoltefuss, Jürgen, Ing. grad.**
**Parkstrasse 20**
**D-5657 Haan 2 (DE)**
Erfinder : **Müller, Lutz, Dr.**
**Kronprinzenallee 111**
**D-5600 Wuppertal 1 (DE)**
Erfinder : **Krause, Hans-Peter, Dr.**
**Wilkhausstrasse 107**
**D-5600 Wuppertal 1 (DE)**
Erfinder : **Sitt, Rüdiger, Dr.**
**Am Jagdhaus 116b**
**D-5600 Wuppertal 1 (DE)**

**EP 0 034 784 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Derivate des 3,4,5-Trihydroxypiperidins, Verfahren zu ihrer Herstellung sowie ihre Verwendung als Arzneimittel, beispielsweise als Mittel gegen Prädiabetes, Gastritis, Obstipation, Infektionen des Gastro-Intestinal-Traktes, Meteorismus, Flatulenz, Karies, Atherosklerose, Hypertension und insbesondere gegen Diabetes, Hyperlipämie und Adipositas sowie in der Tierernährung zur Beeinflussung des Fleisch-Fett-Verhältnisses zugunsten des Fleischanteils.

Die erfindungsgemäßen Verbindungen entsprechen der allgemeinen Formel I

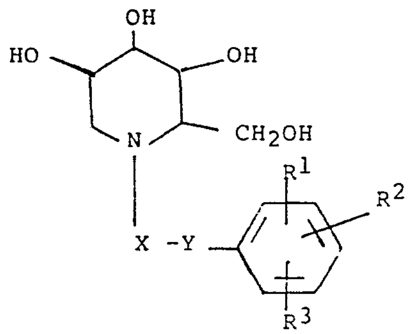

(I)

in der

X einen gesättigten oder ungesättigten Kohlenwasserstoffrest mit 2 bis 10 C-Atomen darstellt,

Y Sauerstoff oder Schwefel bedeutet und

$R^1$, $R^2$, $R^3$ gleich oder verschieden sind und unabhängig voneinander Wasserstoff ; Halogen ; Alkyl mit bis zu 8 C-Atomen ; Phenyl ; CN ; $—OR_4$ mit

$R_4$ = H oder $C_1$-$C_8$-Alkyl ; $—NHR_5$ mit

$R_5$ = H oder Acetyl ; $—COR_6$ mit

$R_6$ = H, $OR_7$ ($R_7$ = H oder $C_1$-$C_8$ Alkyl), $NR_8R_9$ ($R_8$, $R_9$ gleich oder verschieden, = H, $C_1$-$C_7$-Alkyl oder Phenyl),

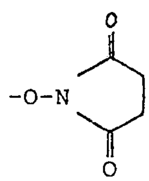 oder 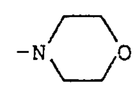 ; oder

$—CH_2R_{10}$ mit $R_{10}$ = OH, CN, $CH_2NH_2$ oder $CH_2—O—C_2H_5—OCH_3$ bedeuten, mit der Maßgabe, daß $R^1$, $R^2$ und $R^3$ nicht gleichzeitig Wasserstoff sein können,

X bedeutet vorzugsweise einen gesättigten oder einodermehrfach ungesättigten Alkylrest mit 2 bis 5 C-Atomen.

Es wurde gefunden, daß die neuen Verbindungen der Allgemeinen Formel I potente Inhibitoren für α-Glucosidasen, insbesondere für Disaccharidasen sind. Daher sind die neuen Verbindungen wertvolle Mittel zur Beeinflussung einer Vielzahl von Stoffwechselvorgängen und bereichern somit den Arzneimittelschatz.

Die erfindungsgemäßen Verbindungen lassen sich nach verschiedenen an sich bekannten Verfahren herstellen. Ein Verfahren ist dadurch gekennzeichnet, daß man eine Verbindung der Formel II

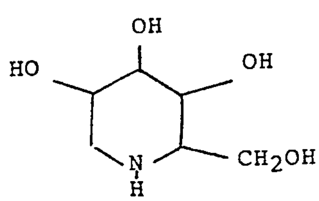

(II)

mit Alkylierungsmitteln der Allgemeinen Formel III

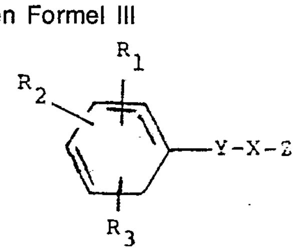

(III)

worin

Z eine in Alkylierungsmitteln übliche funktionelle Gruppe, beispielsweise einen Halogenid- oder einen Sulfonsäureesterrest bedeutet und $R^1$, $R^2$, $R^3$, Y und X die oben angegebene Bedeutung haben, umsetzt.

Setzt man z. B. 1-Desoxynojirimycin und 2-Phenoxyethylbromid miteinander um, so läßt sich der Reaktionsablauf wie folgt wiedergeben.

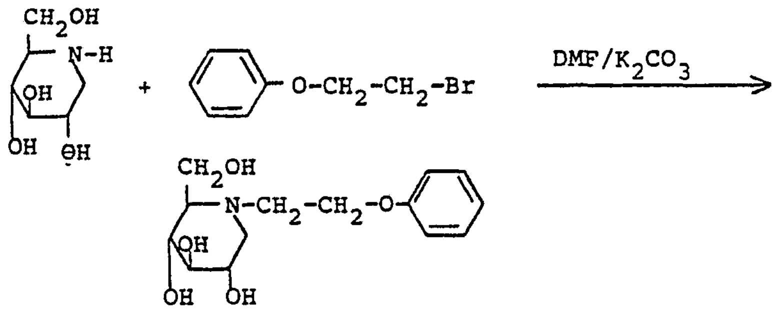


Ein weiteres Verfahren ist dadurch gekennzeichnet, daß man Verbindungen der Formel II mit Aldehyden der Allgemeinen Formel IV

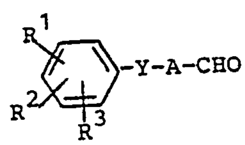
(IV)

worin

A einen gesättigten oder einen ein- oder mehrfach ungesättigten aliphatischen Rest mit einer gegenüber dem Rest X um 1 C-Atom kleineren Kohlenstoffzahl darstellt und $R^1$, $R^2$, $R^3$ und Y die oben angegebene Bedeutung haben, in Gegenwart eines Wasserstoffdonors nach der Verfahrensweise der reduktiven Alkylierung umsetzt.

Setzt man beispielsweise 1-Desoxynojirimycin mit β-Phenoxy-propionaldehyd in Gegenwart von Natriumcyanoborhydrid als Wasserstoffdonor um, so läßt sich der Reaktionsablauf wie folgt wiedergeben.

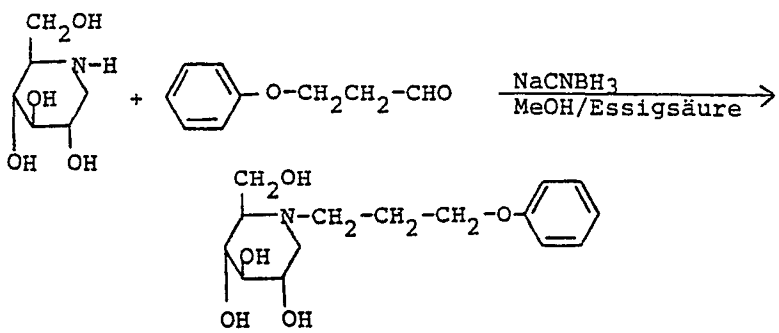


Die verwendeten Ausgangsstoffe sind zum größten Teil bekannt oder können nach bekannten Verfahren hergestellt werden. So ist 1-Desoxynojirimycin z. B. aus EP-A-947 bekannt und 4-Phenoxy-trans-buten-2-yl-bromid und analoge Verbindungen lassen sich nach a. Lüttringhaus, G. v. Sääf und K. Hauschild, B. 71, 1 677 (1 938) herstellen.

Die Umsetzung des Piperidinderivates 1-Desoxynojirimicin II mit den Verbindungen III wird in polaren, protischen oder aprotischen Lösungsmitteln zweckmäßigerweise in Gegenwart eines säurebindenden Mittels bei Temperaturen zwischen 0 °C und Siedetemperatur des Lösungsmittels durchgeführt. Bevorzugt arbeitet man in DMF in Gegenwart von Kaliumcarbonat.

Für die reduktive Alkylierung kann man als Wasserstoffdonor katalytisch erregten Wasserstoff verwenden. Als Katalysator kommt vor allem Raney-Nickel in Frage, es können aber auch Edelmetallkatalysatoren Verwendung finden. Die Reaktion wird im allgemeinen bei Drucken zwischen $10^5$ und $150 \cdot 10^5$ Pa $H_2$-Druck und Temperaturen zwischen 20 und 150 °C durchgeführt. Als Lösungsmittel werden protische, polare Lösungsmittel, besonders Alkohole bevorzugt.

Als Wasserstoffdonor-Reduktionsmittel werden auch Alkalimetallcyanoborhydride, Dialkylaminoborane und Alkalimetallborhydride verwendet. Besonders bevorzugt in dieser Verfahrensvariante ist die

Verwendung von Natrium cyanoborhydrid. Die Reaktion wird im allgemeinen bei Raumtemperatur durchgeführt. Es kann aber auch günstig sein, auf Rückflußtemperatur zu erhitzen.

Das Verfahren wird üblicherweise in einem inerten Lösungsmittel durchgeführt. Obwohl wasserfreie aprotische Lösungsmittel eingesetzt werden können (z. B. Tetrahydrofuran, wenn das Reduktionsmittel Morpholinoboran ist), wird gewöhnlich doch ein protisches Lösungsmittel verwendet. Als solches eignet sich besonders ein niederes Alkanol. Es kann aber auch Wasser oder ein wäßriges niedriges Alkanol (z. B. wäßriges Methanol oder Ethanol) oder andere wäßrige Lösungsmittelsysteme, wie z. B. wäßriges Dimethylformamid, wäßriges Hexamethylphosphorsäuretriamid, wäßriges Tetrahydrofuran oder wäßriger Ethylenglycoldimethylether verwendet werden.

Das Verfahren wird gewöhnlich in einem pH-Bereich von 1 bis 11 durchgeführt, bevorzugt ist ein pH-Bereich zwischen 4 und 7.

Die erfindungsgemäßen Inhibitoren eignen sich als Therapeutika für folgende Indikationen.

Prädiabetes, Gastritis, Obstipation, Infektionen des Gastrointestinal-Traktes, Meteorismus, Flatulenz, Karies, Atherosklerose, Hypertension und besonders Adipositas, Diabetes und Hyperlipämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt. So kann es beispielsweise zweckmäßig sein, erfindungsgemäße Saccharase-Inhibitoren mit bereits bekannten Amylase-Inhibitoren zu kombinieren.

Vorteilhaft sind in manchen Fällen auch Kombinationen der erfindungsgemäßen Inhibitoren mit bekannten oralen Antidiabetica (β-cytotrope Sulfonylharnstoffderivate und/oder blutzuckerwirksame Biguanide) sowie mit blutlipidsenkenden Wirkstoffen wie Clofibrat, Nicotinsäure, Cholestyramin und anderen.

Die Verbindungen können ohne Verdünnung, z. B. als Pulver oder in einer Gelatinehülle oder in Kombination mit einem Trägerstoff in einer pharmazeutischen Zusammensetzung appliziert werden.

Pharmazeutische Zubereitungen können eine größere oder kleinere Menge des Inhibitors enthalten, z. B. 0,1 % bis 99,5 %, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxische inerte und pharmazeutisch-verträgliche Formulierungshilfsmittel enthalten kann. Solche pharmazeutischen Zubereitungen liegen vorzugsweise in Form von Dosierungseinheiten vor, d. h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen, Haupt- und Nebenmahlzeiten am Tage verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännichen Urteils und unter Beachtung des Alters, des Gewichtes und des Zustandes des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, wie z. B. Pulver, Tabletten, Dragees, Kapseln, Granulate, Suspensionen, Lösungen und dergleichen.

Pulver wird durch Zerkleinerung der Substanz in einer geeigneten Größe und Vermischen mit einem ebenfalls zerkleinerten pharmazeutischen Trägerstoff hergestellt. Obgleich ein eßbares Kohlenhydrat, wie z. B. Stärke, Lactose, Saccharose oder Glucose normalerweise zu diesem Zwecke Verwendung findet und auch hier benutzt werden kann, ist es wünschenswert, ein nicht metabolisierbares Kohlenhydrat, wie z. B. ein Cellulosederivat, zu benutzen.

Süßmittel, Geschmackszusätze, Konservierungsstoffe, Dispergiermittel und Färbemittel können auch mitverwendet werden.

Die Kapseln können durch Zubereitung der oben beschriebenen Pulvermischung und durch Füllung bereits gebildeter Gelatinehüllen hergestellt werden. Die Pulvermischung kann man vor dem Füllvorgang mit Gleitmitteln, wie z. B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z. B. Agar-Agar, Calciumcarbonat oder Natriumcarbonat, versetzen, um bei Einnahme der Kapsel die Zugänglichkeit des Inhibitors zu verbessern.

Die Anfertigung der Tabletten erfolgt z. B. durch Herstellung einer Pulvermischung, grob oder feinkörnig, und Hinzufügung eines Gleitmittels und Desintegrators. Aus dieser Mischung formt man Tabletten. Eine Pulvermischung bereitet man vor durch Mischung der Substanz, welche in geeigneter

Weise zerkleinert wurde und ergänzt ein Verdünnungsmittel oder eine andere Trägersubstanz, wie oben beschrieben. Gegebenenfalls fügt man ein Bindemittel hinzu : z. B. Carboxymethylcellulose, Alginate, Gelatine oder Polyvinylpyrrolidone, einen Lösungsverzögerer, wie z. B. Paraffin, einen Resorptionsbeschleuniger, wie z. B. ein quarternäres Salz und/oder ein Adsorptionsmittel, wie z. B. Bentonit, Kaolin oder Dicalciumphosphat. Die Pulvermischung kann granuliert werden zusammen mit einem Bindemittel, wie z. B. Sirup, Stärkepaste, Akazienschleim, oder Lösungen aus Zellulose- oder Polymerenmaterialien. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den tablettenbildenden Düsen stecken bleiben, kann man sie mit einem Gleitmittel versetzen, wie z. B. Stearinsäure, Stearatsalz, Talkum oder Mineralöl. Diese gleitfähig gemachte Mischung wird dann in Tablettenform gepreßt. Die Wirkstoffe können auch mit freifließenden inerten Trägerstoffen vereinigt werden und direkt in Tablettenform gebracht werden unter Auslassung der Granulat- oder Zerstückelungsschritte. Man kann das Produkt mit einer klaren oder opaken Schutzhülle versehen, z. B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z. B. Lösungen, Syrup und Elixire, lassen sich in Dosierungseinheiten herstellen, so daß eine bestimmte Menge Präparat eine bestimmte Menge Wirkstoff enthält. Syrup kann so hergestellt werden, daß der Wirkstoff in einer wäßrigen Lösung, welche geeignete Geschmacksstoffe enthält, gelöst wird ; Elixire werden unter Verwendung nichttoxischer, alkoholischer Trägerstoffe erhalten. Suspensionen kann man durch Dispergieren der Verbindung in einem nichttoxischen Trägerstoff darstellen. Lösungsvermittler und Emulgiermittel, wie z. B. äthoxylierte Isostearylalkohole und Polyoxyäthylensorbitester, Konservierungsmittel, geschmacksverbessernde Zusätze wie z. B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z. B. durch Einhalten des Wirkstoffes in Polymerensubstanzen, Wachse oder dergleichen.

Zusätzlich zu den oben erwähnten pharmazeutischen Zusammensetzungen lassen sich auch diese Wirkstoffe enthaltende Lebensmittel herstellen ; beispielsweise Zucker, Brot, Kartoffelprodukte, Fruchtsaft, Bier, Schokolade und andere Konfektartikel, und Konserven, wie z. B. Marmelade, wobei zu diesen Produkten eine therapeutischwirksame Menge mindestens eines der erfindungsgemäßen Inhibitoren gegeben wurde.

Die unter Verwendung der erfindungsgemäßen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemäßen Inhibitoren weisen weiterhin die Eigenschaft auf, in Tieren das Verhältnis des Anteils an unerwünschtem Fett zum Anteil des erwünschten fettarmen Fleisches (mageres Fleisch) zugunsten des mageren Fleisches in hohem Maße zu beeinflussen. Dies ist von besonderer Bedeutung für die Aufzucht und Haltung von landwirtschaftlichen Nutztieren, z. B. in der Schweinemast, aber auch von erheblicher Bedeutung für die Aufzucht und Haltung von sonstigen Nutztieren und Ziertieren. Die Verwendung der Inhibitoren kann weiterhin zu einer erheblichen Rationalisierung der Fütterung der Tiere führen, sowohl zeitlich, mengenmäßig wie auch qualitätsmäßig. Da sie eine gewisse Verzögerung der Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemäßen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zur Einsparung von Futtereiweiß eingesetzt werden können, seien beispielsweise folgende Nutz- und Ziertiere genannt : Warmblüter wie Rinder, Schweine, Pferde, Schafe, Ziegen, Katzen, Hunde, Kaninchen, Pelztiere, z. B. Nerze, Chinchilla, andere Ziertiere, z. B. Meerschweinchen und Hamster, Labor- und Zootiere, z. B. Ratten, Mäuse, Affen usw. ; Geflügel, z. B. Broiler, Hühner, Gänse, Enten, Truthähne, Tauben, Papageien und Kanarienvögel und Kaltblüter, wie Fische, z. B. Karpfen und Reptilien, z. B. Schlangen.

Die Menge der Wirkstoffe, die ten Tieren zur Erreichung des gewünschten Effektes verabreicht wird, kann wegen der günstigen Eigenschaften der Wirkstoffe weitgehend variiert werden. Sie liegt vorzugsweise bei etwa 0,1 mg bis 1,0 g insbesondere 1 bis 100 mg/kg Futter pro Tag. Die Dauer der Verabreichung kann von wenigen Stunden oder Tagen bis zu mehreren Jahren betragen. Die passende Menge wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jeden Fachmann leicht zu ermitteln.

Die erfindungsgemäßen Wirkstoffe werden den Tieren nach den üblichen Methoden verabreicht. Die Art der Verabreichung hängt insbesondere von der Art, dem Verhalten und dem Allgemeinzustand der Tiere ab. So kann die Verabreichung einmal oder mehrmals täglich, in regelmäßigen oder unregelmäßigen Abständen, oral erfolgen. Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Die Wirkstoffe können als reine Stoffe oder in formulierter Form verabreicht werden, wobei die formulierte Form sowohl als Premix, also in Mischung mit nichttoxischen inerten Trägerstoffen beliebiger Art, als auch als Teil einer Gesamtration in Form eines Beifutters bzw. als Mischungsbestandteil eines alleinigen Mischfutters zu verstehen ist. Mit eingeschlossen ist auch die Applikation geeigneter Zubereitungen über das Trinkwasser.

Die Wirkstoffe können gegebenenfalls in formulierter Form auch zusammen mit anderen Nähr- und Wirkstoffen, z. B. Mineralsalzen, Spurenelementen, Vitaminen, Eiweißstoffen, Energieträgern (z. B. Stärke, Zucker, Fette), Farbstoffen und/oder Geschmacksstoffen oder anderen Futterzusatzstoffen, wie z. B. Wachstumsförderern, in geeigneter Form verabreicht werden. Die Wirkstoffe können den Tieren vor, während oder nach der Nahrungsaufnahme gegeben werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwassers zugegeben werden.

Die Wirkstoffe können nach üblichen Methoden durch einfaches Mischen als reine Stoffe, vorzugsweise in fein verteilter Form oder in formulierter Form in Mischung mit eßbaren, nichttoxischen Trägerstoffen, gegebenenfalls auch in Form eines Premix oder eines Futterkonzentrates, dem Futter und/oder dem Trinkwasser beigefügt werden.

Das Futter und/oder Trinkwasser kann beispielsweise die erfindungsgemäßen Wirkstoffe in einer Konzentration von etwa 0,001 bis 5,0 %, insbesondere 0,02 bis 2,0 % (Gewicht) enthalten. Die optimale Höhe der Konzentration des Wirkstoffs im Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Eiweißstoffen, einschließlich Vitaminen und Mineralstoffen enthalten. Das Futter kann sich beispielsweise zusammensetzen aus pflanzlichen Stoffen. z. B. Ölkuchenschroten, Getreideschroten, Getreidenebenprodukten, aber auch aus Heu, Gärfutter, Rüben und anderen Futterpflanzen, aus tierischen Stoffen, z. B. Fleich- und Fischprodukte, Knochenmehl, Fette, Vitamine, z. B. A, D, E, K und B-Komplex sowie spezielle Proteinquellen, z. B. Hefen sowie bestimmte Aminosäuren und Mineralstoffen und Spurenelementen, wie z. B. Phosphor und Eisen, Zink, Mangan, Kupfer, Kobalt, Jod usw.

Premixe können vorzugsweise etwa 0,1 bis 50 %, insbesondere 0,5 bis 5,0 % (Gewicht) eines erfindungsgemäßen Wirkstoffs neben beliebigen eßbaren Trägerstoffen und/oder Mineralsalzen, z. B. kohlensaurem Futterkalk enthalten und werden nach den üblichen Mischmethoden hergestellt.

Mischfutter enthaltend vorzugsweise 0,001 bis 5,0 %, insbesondere 0,02 bis 2,0 % (Gewicht) eines erfindungsgemäßen Wirkstoffs neben den üblichen Rohstoffkomponenten eines Mischfutters, z. B. Getreideschrote oder -nebenprodukte, Ölkuchenschrote, tierisches Eiweiß, Mineralien, Spurenelemente und Vitamine. Sie können nach den üblichen Mischmethoden hergestellt werden.

Vorzugsweise in Premixen und Mischfuttermitteln können die Wirkstoffe gegebenenfalls auch durch ihre Oberfläche bedeckenden geeigneten Mittel, z. B. mit nichttoxischen Wachsen oder Gelatine vor Luft, Licht und/oder Feuchtigkeit geschützt werden.

Beispiel für die Zusammensetzung eines fertigen Mischfutters für Geflügel, das einen erfindungsgemäßen Wirkstoff enthält :

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Eine Vitamin-Mineral-Mischung kann z. B. bestehen aus :

6 000 I. E. Vitamin A, 1 000 I. E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nicotinsäure, 200 mg Cholinchlorid, 200 g $MnSO_4 \times H_2O$, 140 mg $ZnSO_4 \times 7\ H_2O$, 100 mg $FeSO_4 \times 7\ H_2O$ und 20 mg $CuSO_4 \times 5\ H_2O$.

Der Wirkstoff-Premix enthält einen erfindungsgemäßen Wirkstoff in der gewünschten Menge, z. B. 1 600 mg und zusätzlich 1 g DL-Methionin sowie so viel Sojabohnenmehl, daß 3,2 g Premix entstehen.

Beispiel für eine Zusammensetzung eines Schweinemischfutters, das einen Wirkstoff der allgemeinen Formel I enthält :

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapiokamehl, 38 g Bierhefe, 50 g Vitamin-Mineral-Mischung für Schweine (Zusammensetzung, z. B. wie beim Kükenfutter), 30 g Leinkuchenmehl, 30 g Maiskleberfutter, 10 g Sojaöl, 10 g Zuckerrohrmelasse und 2 g Wirkstoff-Premix (Zusammensetzung z. B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder aber auch in beliebigen Mischungen untereinander verwendet werden.

Saccharase-Inhibitionstest in vitro

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100 %-Wert) des Inhibitors. Als Substrat, welches die Spezifität des Inhibitionstestes bestimmt, dient dabei eine praktisch Glucosefreie Saccharose (Glucose < 100 ppm) ; die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als diejenige inhibitorische Aktivität, welche in einem definierten Testansatz eine vorgegebene saccharolytische Aktivität um eine Einheit (Saccharase-Einheit = SE) reduziert ; die Saccharase-Einheit ist dabei als diejenige Enzymaktivität definiert, welche unter vorgegebenen Bedingungen ein µmol Saccharose pro Minute spaltet und damit zur Freisetzung von je ein µmol Glucose, welche im Test bestimmt wird, und Fructose, welche im Test nicht erfaßt wird, führt.

Der intestinale Disaccharidasen-Komplex wird aus Schweinedünndarm-Mucosa durch tryptische Verdauung, Fällung aus 66 % Äthanol bei – 20 °C, Aufnehmen des Präcipitates in 100 mM Phosphat-Puffer, pH 7,0 und abschließende Dialyse gegen denselben Puffer gewonnen.

10 µl einer Probelösung, die so angesetzt ist, daß die Extinktion des Testansatzes mindestens 10 %, jedoch nicht mehr als 25 % unter der des 100 %-Wertes liegt, werden mit 100 µl einer Verdünnung des intestinalen Disaccharidasen-Komplexes in 0,1 M Maleinat-Puffer, pH 6,25, versetzt und für 10 Minuten bei 37 °C vorinkubiert. Die Verdünnung des Disaccharidasen-Komplexes ist auf eine Aktivität von 0,1 Se/ml einzustellen.

Anschließend wird die saccharolytische Reaktion durch Zugabe von 100 µl einer 0,4 M Lösung von Saccharose (« SERVA 35 579 ») in 0,1 M Maleinat-Puffer, pH 6,25 gestartet und nach einer Inkubationsdauer von 20 Minuten bei 37 °C durch die Zugabe von 1 ml Glucose-Dehydrogenase-Reagenz (1 Fläschchen Glucose-Dehydrogenase-Mutarotase-Gemisch lyophiliert (« MERCK 14 053 ») und 331,7 mg β-Nicotinamid-adenin-dinucleotid (freie Säure, « BOEHRINGER » Reinheitsgrad I) in 250 ml 0,5 M Tris-Puffer, pH 7,6 gelöst) abgestoppt. Zum Nachweis der Glucose wird 30 Minuten bei 37 °C inkubiert und schließlich bei 340 nm gegen einen Reagenzienblank (mit Enzym, jedoch ohne Saccharose) photometriert.

Die Berechnung der Hemmaktivität von Inhibitoren ist dadurch erschwert, daß schon geringfügige Änderungen im Testsystem, beispielsweise ein geringfügig von Bestimmung zu Bestimmung variierender 100 %-Wert, von nicht mehr zu vernachlässigendem Einfluß auf das Testergebnis sind. Man umgeht diese Schwierigkeiten, indem man bei jeder Bestimmung einen Standard mitlaufen läßt ; als Standard dient ein Saccharase-Inhibitor der Formel $C_{25}H_{43}O_{18}N$, welcher eine spezifische Hemmaktivität von 77 700 SIE/g aufweist und bei eingesetzten Mengen von 10 bis 20 ng im Test zu einer Hemmung von oben spezifizierter Größenordnung führt. Bei Kenntnis der Differenz der Extinktionen bei 340 nm von 100 %-Wert und durch Standard gehemmtem Ansatz läßt sich aus der Extinktions-differenz von 100 %-Wert und durch die Probelösung gehemmtem Ansatz unter Berücksichtitung der eingesetzten Menge an Inhibitor in bekannter Weise dessen spezifische Hemmaktivität errechnen, ausgedrückt in Saccharase-Inhibitor-Einheiten pro Gramm (SIE/g).

Beispiel 1

N-β-Phenoxyethyl-1-desoxynojirimycin

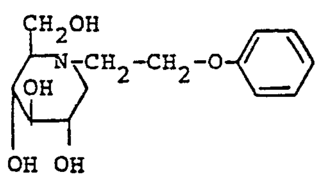

Eine Suspension von 9,7 g Desoxynojirimycin und 12,4 g gepulvertem Kaliumcarbonat in 100 ml absolutem Dimethylformamid wird mit 15,7 g β-Phenoxyethylbromid 5 Stunden bei 90-100 °C gerührt. Es wird abgekühlt und abgesaugt. Das Filtrat wird bei 60° Badtemperatur am Rotationsverdampfer eingeengt. Der Eindampfrückstand wird in wenig Wasser heiß gelöst, 18 Stunden bei 5 °C gehalten, die erhaltenen Kristalle abgesaugt und mit Eiswasser gewaschen. Man erhält 10,5 g eines Kristallisates vom Schmelzpunkt 146 °C.

Beispiel 2

N-(5-Phenoxy-pentyl)-1-desoxynojirimycin

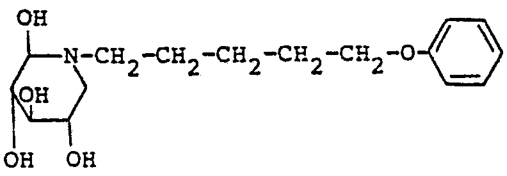

4,6 g 1-Desoxynojirimycin werden mit 6,2 g gepulvertem Kaliumcarbonat und 11 g 5-Phenoxypentylbromid 5 Stunden bei 100 °C gerührt. Es wird abgekühlt und abgesaugt. Das Filtrat wird bei 70 °C am Rotationsverdampfer eingeengt. Der erhaltene Eindampfrückstand wird in ca. 300 ml Ethanol gelöst. Es wird nach Zugabe von Tonsil als Filtrierhilfsmittel filtriert und eingeengt. Das halbfeste Produkt wird mit Acetonitril verrührt, abgesaugt und mit Acetonitril und Wasser gewaschen. Man erhält 6,35 g eines farblosen Produktes vom Schmelzpunkt 138-39 °C.

Analog wurden hergestellt :

Beispiel 3

N-(4-Phenoxybutyl)-1-desoxynojirimycin vom Schmelzpunkt ab 110 °C.

Beispiel 4

N-[β-(2,6-Dimethyl-phenoxy)-ethyl]-1-desoxynojirimycin vom Schmp. : 155-56 °C.

Beispiel 5

N-[γ-(2,6-Dimethoxyphenoxy)-propyl]-1-desoxynojirimycin vom Schmp. : 128 °C.

Beispiel 6

N-[β-(2,4-Dichlorphenoxy)-ethyl]-1-desoxynojirimycin vom Schmelzpunkt 175-76 °C.

Beispiel 7

N-(γ-Phenoxypropyl)-1-desoxynojirimycin vom Schmp. : 152 °C.

Beispiel 8

N-(4-Phenoxy-trans-buten-2-yl)-1-desoxynojirimycin-hydrat

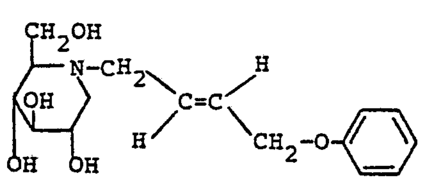 $\cdot H_2O$

Eine Suspension von 3,6 g 1-Desoxynojirimycin und 4,55 g gemahlenem Kaliumcarbonat in 40 ml absolutem Dimethylformamid wird mit 6,2 g 1-Phenoxy-4-brom-trans-buten-2 versetzt und 5 Stunden bei 100 °C gerührt. Es wird abgekühlt und vom Salz abgesaugt. Das Filtrat wird bei 60 °C eingeengt und der Eindampfrückstand mit wenig Wasser verrührt. Das entstandene Festprodukt wird abgesaugt und mit wenig Wasser und Isopropanol gewaschen. Nach dem Umkristallisieren aus Wasser erhält man 3,1 g fast farblose Kristalle vom Schmelzpunkt 120 °C.

Analog wurden hergestellt :

Beispiel 9

N-(4-p-Methoxyphenyloxy-trans-buten-2-yl)-1-desoxynojirimycin

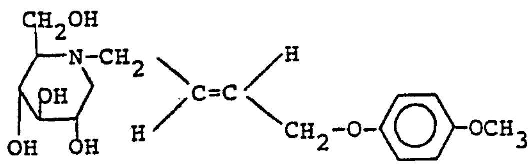

Fp. : 163-166 °C.
Herstellung des Ausgangsmaterials :
Das Ausgangsmaterial 1-p-Methoxyphenyloxy-4-brom-trans-buten-2 wurde nach A. Lüttinghaus et. al. [B. 71, 1 677 (1938)] aus 1,4-Dibrom-trans-buten-2 und p-Methoxyphenol hergestellt.
Fp. : 58 °C.

Beispiel 10

N-[4-(4-Carbethoxyphenyloxy)-buten-2-yl]-1-desoxynojirimycin

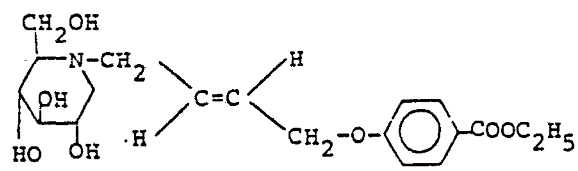

Beispiel 11

N-[β-(4-Methoxy-phenoxy)-ethyl]-1-desoxynojirimycin vom Schmp. : 175-78 °C.

Beispiel 12

N-[β-(4-Chlorphenoxy)-ethyl]-1-desoxynojirimycin vom Schmp. : 156-57 °C.

Beispiel 13

N-[β-(4-Cyano-phenoxy)-ethyl]-1-desoxynojirimycin vom Schmp. : 125 °C.

Beispiel 14

N-[β-(3-Methylphenoxy)-ethyl]-1-desoxynojirimycin vom Schmp. : 132-34 °C.

Beispiel 15

a) Herstellung von 2-Phenylthioethylbromid

33 g Thiophenol werden in einer Lösung von 6,9 g Natrium in 120 ml Ethanol gelöst und in 129 ml 1,2-Dibromethan getropft. Es wird 1 Stunde unter Rückfluß erhitzt, abgekühlt und mit 250 ml Ether versetzt. Es wird vom ausgefallenen Salz abgesaugt. Das Filtrat wird eingeengt und der Eindampfrückstand fraktioniert. Man erhält 55,4 g farbloses Öl vom Siedepunkt 125-130 °C bei $1,6 \cdot 10^3$ Pa.

b) N-(β-Phenylthioethyl)-1-desoxynojirimycin

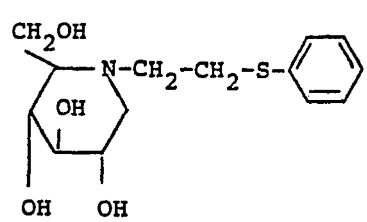

19,4 g 1-Desoxynojirimycin werden mit 24,8 g gepulvertem Kaliumcarbonat und 33,9 g 2-Phenylthioethylbromid in 200 ml absolutem DMF 8 Stunden bei 90-100 °C gerührt. Es wird abgekühlt, abgesaugt und das Filtrat bei 60 °C am Rotationsverdampfer eingeengt. Der ölige Eindampfrückstand wird über eine 120 cm lange und 6 cm weite Säule gereinigt, die als stationäre Phase Cellulose und als mobile Phase zuerst Aceton, dann 95 %iges Aceton enthält. Die sauberen Fraktionen werden vereint und eingeengt. Es

wird aus Isopropanol umkristallisiert. Man erhält 16,3 g einer farblosen Substanz vom Schmp. : 121-123 °C.

Analog wurde erhalten :

Beispiel 16

N-[β-(4-Methylphenylthio)-ethyl]-1-desoxynojirimycin vom Schmp. : 126-27 °C.

Beispiel 17

a) Herstellung von 4-(3-Methylphenylthio)-buten-2-yl-bromid

Eine Lösung von 2,76 g Natrium in 80 ml absolutem Methanol wird mit 14,9 g 3-Methylthiophenol versetzt und anschließend bei 30-35 °C in eine Lösung von 85,6 g 1,4-Dibrombuten-2 in 100 ml Ether getropft. Es wird 30 Minuten zum Sieden erhitzt, abgekühlt und abgesaugt. Das Filtrat wird bei 30 °C Badtemperatur am Rotationsverdampfer eingeengt und der Eindampfrückstand bei 110 °C Badtemperatur und $1{,}3 \cdot 10^2$ Pa weitgehend vom überschüssigen 1,4-Dibrombuten-2 befreit. Der erhaltene Destillationsrückstand von 22 g wird ohne weitere Reinigung umgesetzt.

b) N-[4-(3-Methylphenylthio)-buten-2-yl]-1-desoxynojirimycin

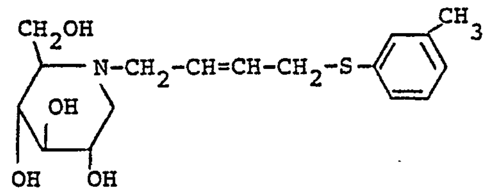

Ein Gemisch von 7,2 g 1-Desoxynojirimycin, 9,1 g gepulvertem Kaliumcarbonat und 22 g 4-(3-Methylphenylthio)-buten-2-yl in 80 ml absolutem DMF wird 7 Stunden bei 100 °C gerührt. Es wird abgekühlt, abgesaugt und eingeengt. Der erhaltene Eindampfrückstand wird auf eine Säule aufgetragen, die als stationäre Phase Cellulose und als mobile Phase Aceton enthält. Das reine Produkt wird mit 95 % Aceton erhalten. Die sauberen Fraktionen werden eingeengt. Es wird mit wenig Ethanol kristallisiert. Man erhält farblose Kristalle vom Schmp. : 106 °C.

Beispiel 18

N-[4-(4-Chlorphenylthio)-buten-2-yl]-1-desoxynojirimycin

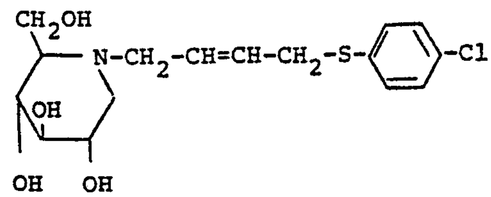

7,2 g 1-Desoxynojirimycin, 9,1 g Kaliumcarbonat und 20,8 g 4-(4-Chlorphenylthio)-buten-2-ylbromid (roh) werden in 80 ml absolutem DMF 6 Stunden bei 100 °C gerührt. Es wird abgekühlt, abgesaugt und eingeengt. Der Eindampfrückstand wird mit Wasser verrieben und das entstandene Festprodukt abgesaugt. Nach Umkristallisation aus Acetonitril mit etwas Isopropanol erhält man 6,7 g farblose Kristalle vom Schmp. : 93-95 °C.

Analog werden hergestellt :

Beispiel 19

N-[4-(tert.-Butylphenylthio)-buten-2-yl]-1-desoxynojirimycin vom Schmp. : 138-40 °C.

Beispiel 20

N-[4-(4-Methylphenylthio)-buten-2-yl]-1-desoxynojirimycin vom Schmp. : ab 83 °C.

Beispiel 21

N-[4-(4-Phenylphenoxy)-buten-2-yl]-1-desoxynojirimycin vom Schmp. : 165-69 °C.

Analog Beispiel 1 wurden hergestellt :

Beispiel 22

N-[β-(4-Acetamidophenoxy)-ethyl]-1-desoxynojirimycin vom Schmp. : 169-170 °C.

Beispiel 23

N-[β-(4-Ethoxycarbonyl-phenoxy)-ethyl]-1-desoxynojirimycin vom Schmp. : 154 °C.

Beispiel 24

N-[β-(4-Formylphenoxy)-ethyl]-1-desoxynojirimycin vom Schmp. : 149 °C.

Beispiel 25

N-[β-(4-Hydroxyphenoxy)-ethyl]-1-desoxynojirimycin vom Schmp. : 149-151 °C.

Beispiel 26

N-[β-(3-Ethoxycarbonylphenoxy)-ethyl]-1-desoxynojirimycin vom Schmp. : 116 °C.

Beispiel 27

N-[4-(4-Acetamidophenoxy)-buten-2-yl]-1-desoxynojirimycinhydrat vom Schmp. : 82 °C.

Beispiel 28

N-[β-(4-Aminomethylphenoxy)-ethyl]-1-deoxynojirimycin

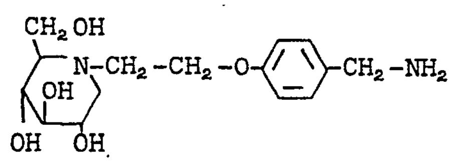

5 g von Beispiel Nr. 13 werden in 200 ml Wasser und 50 ml 25 %igem Ammoniak gelöst und nach Zugabe von Raney-Nickel 2 Stdn. bei 3,5 · $10^5$ Pa hydriert. Es wird vom Katalysator abfiltriert, eingeengt und aus Methanol um kristallisiert. Man erhält 3,0 g farblose Kristalle vom Schmp. : 169 °C.

Beispiel 29

N-[β-(4-Hydroxymethylphenoxy)-ethyl]-1-desoxynojirimycin vom Schmelzpunkt 173-74 °C wird durch Hydrierung der Verbindung aus Beispiel 24 erhalten.

Beispiel 30

N-[4-(4-Aminophenoxy)-but-2-en-yl]-1-desoxynojirimycin

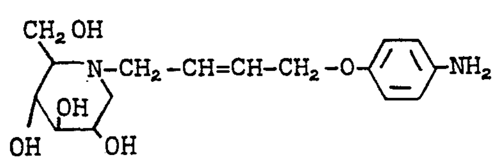

2,6 g Verbindung aus Beispiel 27 werden mit 11 ml halbkonzentrierter Salzsäure 120 Min. bei 80-85 °C gerührt, eingeengt und über einen Kationenaustauscher gereinigt. Man erhält nach Umkristallisation aus Wasser 400 mg farblose Substanz vom Schmp. : 110 °C.

Beispiel 31

N-[β-(4-Aminophenoxy)-ethyl]-1-desoxynojirimycin-dihydrochlorid vom Schmelzpunkt 272 °C unter Zersetzung wird durch salzsaure Hydrolyse der Verbindung aus Beispiel 22 erhalten.

Beispiel 32

N-[β-(4-Hydroxycarbonylphenoxy)-ethyl]-1-desoxynojirimycin

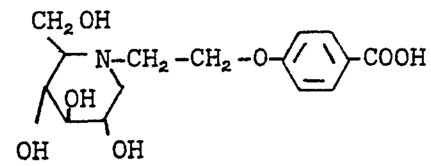

1 g Verbindung aus Beispiel 23 wird in 10 ml 1 N Natronlauge 1 Stde. bei 80 bis 90 °C gerührt. Es wird mit 10 ml 1 N Salzsäure versetzt, eingeengt, mit wenig Wasser verrührt, abgesaugt und mit Wasser gewaschen. Man erhält 0,8 g farblose Substanz vom Schmp. : 235-37 °C.

Beispiel 33

N-[β-(3-Hydroxycarbonylphenoxy)-ethyl]-1-desoxynojirimycin wurde analog Beispiel 32 durch Hydrolyse der Verbindung aus Beispiel 26 erhalten und als Schaum isoliert.

Beispiel 34

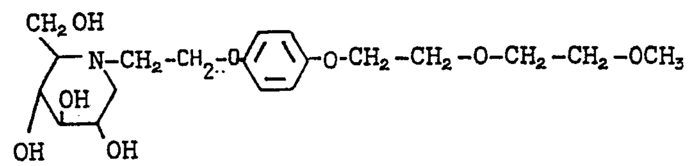

0,23 g Natrium werden in 40 ml absolutem Ethanol gelöst und mit 3 g der Verbindung aus Beispiel 25 versetzt. Es wird eingeengt und nach Zugabe von DMF wieder eingeengt. Der Eindampfrückstand wird in 10 ml abs. DMF gelöst, mit 2,7 g β-Brom-β'-methoxydiäthylester versetzt und 4 Stdn. bei 100 bis 120 °C gerührt. Es wird eingeengt und der Rückstand über eine Aceton/Cellulose-Säule gereinigt. Durch Umkristallisation aus Aceton erhält man 600 mg nahezu farbloser Kristalle vom Schmp. : 118 °C.

Beispiel 35

N-[β-(4-tert.-Butylphenylthio)-ethyl]-1-desoxynojirimycin wurde analog Beispiel 15 aus 1-Desoxynojirimycin und β-(4-tert.-Butylphenylthio)-ethylbromid als farbloses Oel erhalten.

Beispiel 36

Analog Beispiel 17 wurde hergestellt :
N-(4-Phenylthiobut-en-2-yl)-1-desoxynojirimycin vom Schmp. : 117-119 °C.

Beispiel 37

N-[β-(4-Cyanomethylphenoxy)-ethyl]-1-desoxynojirimycin vom Schmp. : 128-32 °C.

Beispiel 38

N-[β-(4-Aminoethylphenoxy)-ethyl]-1-desoxynojirimycin vom Schmelzpunkt 159-62 °C wurde durch Hydrierung der Verbindung aus Beispiel 37 analog Beispiel 28 hergestellt.

Beispiel 39

N-Hydroxysuccinimidester des N-[β-(4-Hydroxycarbonylphenoxy)-ethyl]-1-desoxynojirimycins

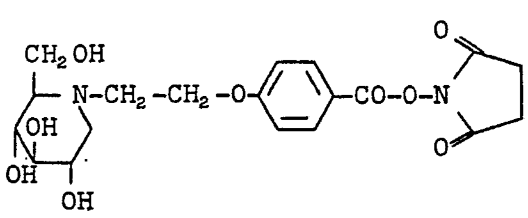

3,27 g Verbindung aus Beispiel 32 werden in 35 ml heißem, absolutem DMF gelöst, auf ca. 25 °C abgekühlt und unter Rühren mit 1,3 g N-Hydroxysuccinimid und 2,3 g Dicyclohexylcarbodiimid versetzt.

Es wird 20 Stunden gerührt, vom ausgefallenen Dicyclohexylharnstoff abfiltriert und bei 30 °C Badtemperatur eingeengt. Der Eindampfrückstand wird in ca. 25 ml warmen Wasser aufgenommen, schnell von Rückstand filtriert und im Eisbad kristallisiert. Man erhält 2,2 g farblose Kristalle vom Schmelzpunkt 137-139 °C unter Zersetzung.

Beispiel 40

N-[β-(4-Carbamoyl-phenoxy)-ethyl]-1-desoxynojirimycin

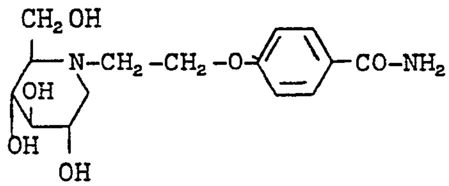

6 g der Verbindung aus Beispiel 39 werden in 60 ml 25 %igem Ammoniak eingetragen und 24 Stunden gerührt. Es wird eingeengt, in wenig Wasser aufgenommen und auf eine 120 cm lange und 5 cm weite Säule aufgetragen, die als stationäre Phase Cellulose (Avicel Merck) und als mobile Phase wäßriges Aceton enthält. Die Substanz wird mit 90 %igem Aceton erhalten. Nach Umkristallisation aus wenig Wasser erhält man 2,6 g farblose Kristalle vom Schmelzpunkt 183-84 °C.

Analog wurde hergestellt :

Beispiel 41

N-[β-(4-Morpholinocarbonyl-phenoxy)-ethyl]-1-desoxynojirimycin, isoliert als Schaum.

Rf-Wert = 0,595 Rf-Wert für 1-Desoxynojirimycin = 0,135 Fließmittel : Chloroform/Methanol/25 %iger Ammoniak im Volumenverhältnis 6 : 4 : 1. DC-Platten : Kieselgel 60, F 254.

Beispiel 42

N-[2-(4-Phenylcarbamoylphenoxy)-ethyl]-1-desoxynojirimycin

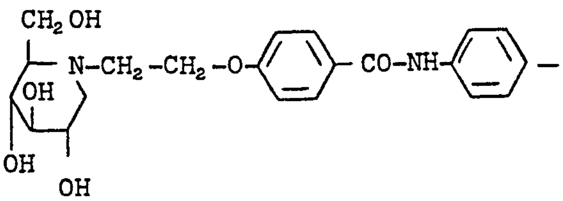

6 g der Verbindung aus Beispiel 39 wird mit 15 ml Anilin 28 Stdn. bei 120 °C Badtemperatur gerührt. Es wird abgekühlt, das ausgefallene Festprodukt mit Essigester verrührt, abgesaugt und mit Essigester gewaschen. Das Festprodukt wird mit 30 ml 1 N Natronlauge verrührt, abgesaugt und mit Wasser gewaschen. Nach Umkristallisation aus DMF/Wasser wurden 3,8 g einer leicht gefärbten Substanz vom Schmelzpunkt 196 °C erhalten.

Beispiel 43

N-[2-(4-Phenylphenoxy)-ethyl]-1-desoxynojirimycin

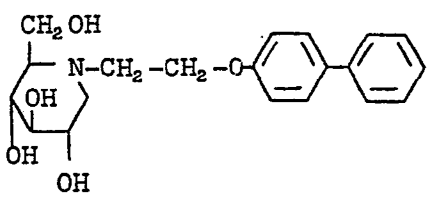

vom Schmp. : 198 °C wird analog Beispiel 1 aus 1-Desoxynojirimycin und 2-(4-Phenylphenoxy)-ethylbromid erhalten.

**Ansprüche**

1. Verbindungen der allgemeinen Formel I

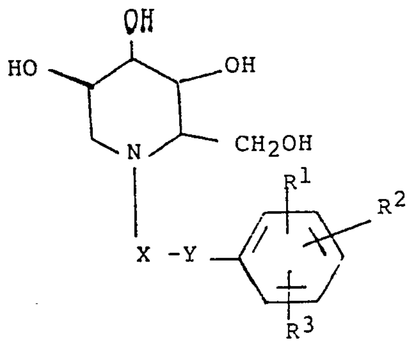

(I)

in der

X einen gesättigten oder ungesättigten Kohlenwasserstoffrest mit 2 bis 10 C-Atomen darstellt,

Y Sauerstoff oder Schwefel bedeutet und

$R^1$, $R^2$, $R^3$ gleich oder verschieden sind und unabhängig voneinander Wasserstoff ; Halogen ; Alkyl mit bis zu 8 C-Atomen ; Phenyl ; CN ; $-OR_4$ mit

$R_4$ = H oder $C_1$-$C_8$-Alkyl ; $-NHR_5$ mit

$R_5$ = H oder Acetyl ; $-COR_6$ mit

$R_6$ = H, $OR_7$ ($R_7$ = H oder $C_1$-$C_8$ Alkyl), $NR_8R_9$ ($R_8$, $R_9$ gleich oder verschieden, = H, $C_1$-$C_7$-Alkyl oder Phenyl),

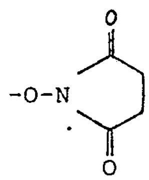 oder 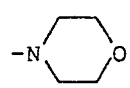 ; oder

$-CH_2R_{10}$ mit $R_{10}$ = OH, CN, $CH_2NH_2$ oder $CH_2-O-C_2H_5-OCH_3$ bedeuten, mit der Maßgabe, daß $R_1$, $R^2$ und $R^3$ nicht gleichzeitig Wasserstoff sein können.

2. Verbindungen nach Anspruch 1, dadurch gekennzeichnet, daß X einen gesättigten oder einen ein- oder mehrfach ungesättigter Alkylrest mit zwei bis fünf C-Atomen bedeutet.

3. N-[β-(4-Ethoxycarbonyl-phenoxy)-ethyl]-1-desoxynojirimycin.

4. Verfahren zur Herstellung der Verbindungen nach Anspruch 1, dadurch gekennzeichnet, daß man in an sich bekannter Weise eine Verbindung der Formel II

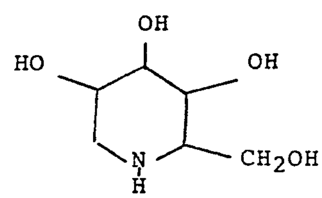

(II)

entweder mit einem Alkylierungsmittel der allgemeinen Formel III

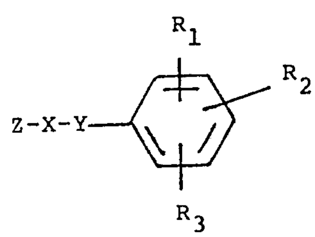

(III)

in der

X, Y, $R_1$, $R_2$, $R_3$ die in Anspruch 1 angegebene Bedeutung haben und

Z eine in Alkylierungsmitteln übliche funktionelle Gruppe bedeutet,

oder nach der Verfahrensweise der reduktiven Alkylierung mit einer Verbindung der allgemeinen Formel IV

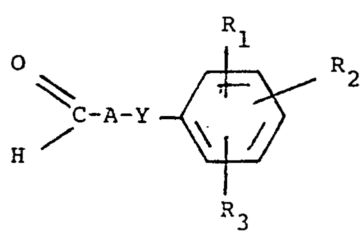

(IV)

in der

Y, $R_1$, $R_2$, $R_3$ die in Anspruch 1 angegebene Bedeutung haben und

A der Bedeutung von X in Anspruch 1 entspricht, jedoch jeweils ein Kohlenstoffatom weniger besitzt, umsetzt.

5. Verfahren nach Anspruch 4, dadurch gekennzeichnet, daß X die in Anspruch 2 angegebene Bedeutung hat bzw. A der Bedeutung von X in Anspruch 2 entspricht, jedoch jeweils ein C-Atom weniger besitzt.

6. Verfahren nach Anspruch 4, dadurch gekennzeichnet, daß man N-[β-(4-Ethoxycarbonyl-phenoxy)-ethyl]-1-desoxynojirimycin herstellt.

7. Arzneimittel gekennzeichnet durch einen Gehalt mindestens einer Verbindung gemäß Anspruch 1, 2 oder 3 und gegebenenfalls pharmazeutisch geeigneten Zusatzstoffen.

8. Tierfuttermittel gekennzeichnet durch einen Gehalt mindestens einer Verbindung nach Anspruch 1, 2 oder 3.

9. Verwendung einer Verbindung gemäß Anspruch 1, 2 oder 3 bei der Tierernährung.

**Claims**

1. Compounds of the general formula

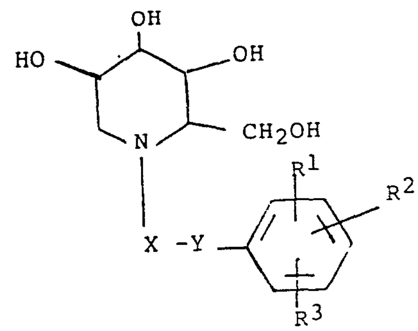

(I)

in which

X represents a saturated or unsaturated hydrocarbon radical with 2 to 10 C atoms;

Y denotes oxygen or sulphur and

$R^1$, $R^2$ and $R^3$ are identical or different and independently of one another denote hydrogen ; halogen ; alkyl with up to 8 C atoms ; phenyl ; CN ; $-OR_4$ in which

$R_4$ = H or $C_1$-$C_8$-alkyl ; $-NHR_5$ in which

$R_5$ = H or acetyl ; $-COR_6$ in which

$R_6$ = H, $OR_7$ ($R_7$ = H or $C_1$-$C_8$ alkyl), $NR_8R_9$ ($R_8$ and $R_9$ are identical or different and = H, $C_1$-$C_7$-alkyl or phenyl),

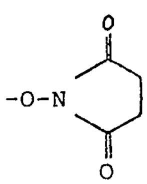 or 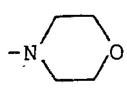 ; or

$-CH_2R_{10}$ in which $R_{10}$ = OH, CN, $CH_2NH_2$ or $CH_2-O-C_2H_5-OCH_3$, with the proviso that $R^1$, $R^2$ and $R^3$ cannot simultaneously be hydrogen.

2. Compounds according to Claim 1, characterised in that X denotes a saturated or mono- or polyunsaturated alkyl radical with two to five C atoms.

3. N-[β-(4-Ethoxycarbonyl-phenoxy)-ethyl]-1-desoxynojirimycin.

4. Process for the preparation of the compounds according to Claim 1, characterised in that a compound of the formula II

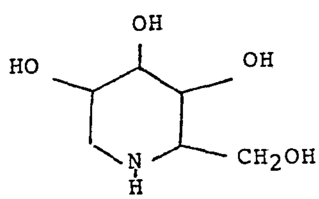

(II)

is reacted in a manner which is in itself known, either with an alkylating agent of the general formula III

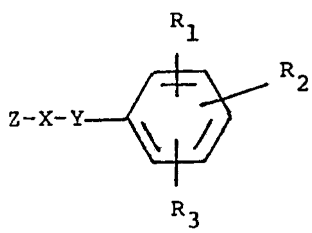

(III)

in which

X, Y, $R_1$, $R_2$ and $R_3$ have the meaning indicated in Claim 1 and

Z denotes a functional group customary in alkylating agents, or in accordance with the procedure of reductive alkylation with a compound of the general formula IV

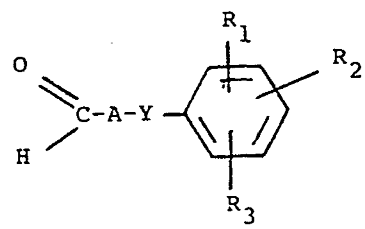

(IV)

in which

Y, $R_1$, $R_2$ and $R_3$ have the meaning indicated in Claim 1 and

A corresponds to the meaning of X in Claim 1, but in case has one less carbon atom.

5. Process according to Claim 4, characterised in that X has the meaning indicated in Claim 2 and A corresponds to the meaning of X in Claim 2, but in each case possesses one less C atom.

6. Process according to Claim 4, characterised in that N-[β-(4-ethoxycarbonyl-phenoxy)-ethyl]-1-desoxynojirimycin is prepared.

7. Medicament, characterised in that it contains at least one compound according to Claim 1, 2 or 3 and optionally pharmaceutically suitable additives.

8. Animal feed, characterised in that it contains at least one compound according to Claim 1, 2 or 3.

9. Use of a compound according to Claim 1, 2 or 3 in animal nutrition.

**Revendications**

1. Composés de formule générale I

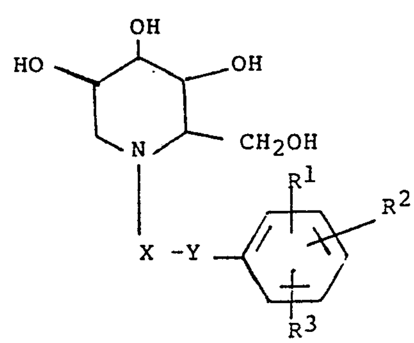

(I)

dans laquelle

X représente un radical d'hydrocarbure saturé ou insaturé contenant 2 à 10 atomes de carbone,

Y représente un atome d'oxygène ou de soufre, et

$R_1$, $R_2$ et $R_3$ sont identiques ou différents et représentent chacun indépendamment l'un de l'autre un atome d'hydrogène ; un atome d'halogène ; un groupe alkyle contenant jusqu'à 8 atomes de carbone ; un groupe phényle ; un groupe CN ; un groupe $-OR_4$ où

$R^4$ = H ou groupe alkyle en $C_1$-$C_8$ ; un groupe $-NHR_5$ où

$R^5$ = H ou groupe acétyle ; un groupe $-COR_6$ où

$R^6$ = H, $OR_7$ ($R_7$ = H ou groupe alkyle en $C_1$-$C_8$), $NR_8R_9$ ($R_8$, $R_9$, identiques ou différents, = H, groupe alkyle en $C_1$-$C_7$ ou groupe phényle),

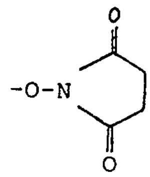 ou 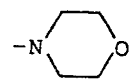 ; ou

un groupe $-CH_2R_{10}$ où $R_{10}$ = OH, CN, $CH_2NH_2$ ou $CH_2-O-C_2H_5-OCH_3$, avec la mesure que $R_1$, $R_2$ et $R_3$ ne peuvent pas être simultanément de l'hydrogène.

2. Composés suivant la revendication 1, caractérisés en ce que X représente un groupe alkyle saturé ou à insaturation simple ou multiple contenant 2 à 5 atomes de carbone.

3. N-[β-(4-éthoxycarbonyl-phénoxy)-éthyl]-1-désoxynojirimycine.

4. Procédé de préparation des composés suivant la revendication 1, caractérisé en ce qu'on fait réagir, de façon connue en soi, un composé de formule II

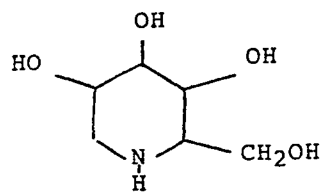

(II)

soit avec un agent d'alkylation de formule générale III

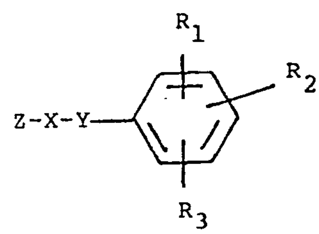

(III)

dans laquelle

X, Y, $R_1$, $R_2$ et $R_3$ ont les significations indiquées dans la revendication 1, et

Z représente un groupe fonctionnel habituellement présent dans des agents d'alkylation,

soit, selon le procédé d'alkylation réductrice, avec un composé de formule générale IV :

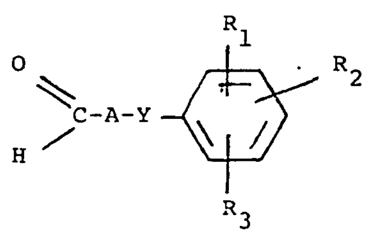

(IV)

dans laquelle

Y, $R_1$, $R_2$ et $R_3$ ont les significations indiquées dans la revendication 1, et

A correspond à la signification de X dans la revendication 1, mais comporte chaque fois un atome de carbone en moins.

5. Procédé suivant la revendication 4, caractérisé en ce que X a la signification indiquée dans la revendication 2 ou A correspond à la signification de X dans la revendication 2, mais comporte chaque fois un atome de carbone en moins.

6. Procédé selon la revendication 4, caractérisé en ce qu'on prépare de la N-[β-(4-éthoxycarbonyl-phénoxy)-éthyl]-1-désoxynojirimycine.

7. Médicament, caractérisé en ce qu'il contient au moins un composé suivant les revendications 1, 2 ou 3 et éventuellement des additifs pharmaceutiquement appropriés.

8. Aliment pour animaux, caractérisé en ce qu'il contient au moins un composé suivant les revendications 1, 2 ou 3.

9. Utilisation d'un composé suivant les revendications 1, 2 ou 3 dans l'alimentation des animaux.